# EUROPEAN PATENT APPLICATION

(11) **EP 3 312 609 A1**
(43) Date of publication of application: **25.04.2018**
(21) Application number: 17195908.3
(22) Date of filing: 11.10.2017
(51) Int. Cl.: G01N 33/50, G01N 21/64

(54) **METHOD FOR ASSESSING IRRITANCY TO BIOLOGICAL SAMPLES**

(30) Priority: 19.10.2016 JP 2016205106
(71) Applicant: Olympus Corporation, Hachioji-shi, Tokyo 192-8507 (JP)
(72) Inventor: Akiyoshi, Ryutaro, Hachioji-shi, Tokyo 192-8507 (JP)
(74) Representative: Gunzelmann, Rainer

(57) **Abstract**

Irritancy of a chemical in the depth direction is assessed while still preserving the three-dimensional structure of a biological sample. A method for assessing irritancy to a biological sample of the present invention is a method for assessing irritancy of a chemical to a three-dimensional biological sample having a plurality of layers in the depth direction and includes: image acquisition step S3 of acquiring, at different depths of the biological sample, fluorescence images of a nuclei of cells in at least one group of a living cell group and a dead cell group in the biological sample exposed to the chemical, the nuclei of cells in at least one group being labeled with a fluorescent marker; and assessment step S4 of calculating the ratio of the number of cells in the at least one cell group existing in the depth direction on the basis of the plurality of fluorescence images.

## Description

### {Technical Field}

The present invention relates to methods for assessing irritancy to biological samples and particularly to a method for assessing irritancy of a chemical to a three-dimensional human skin model.

### {Background Art}

A skin irritation test (RHE method) using a three-dimensional human skin model, which is one of the alternatives to animal tests for assessing the irritancy of various types of chemicals to the human skin, is approved in the testing guidelines of the Organisation for Economic Co-operation and Development (OECD) (refer to Non Patent Literature 1). The RHE method is a method for measuring, according to the MTT test, a cell survival rate in a skin model exposed to a test sample and thereby determining whether or not the chemical is irritant on the basis of the cell survival rate. For example, Patent Literature 1 describes a method for counting the number of living cells by successively acquiring images of the living cells using a camera provided thereabove while carrying a plurality of wells in which test samples are accommodated.

### {Citation List}

### {Patent Literature}

{PTL 1}
Japanese Unexamined Patent Application, Publication No. 2007-24576

### {Non Patent Literature}

{NPL 1}
OECD, "Test No. 439: In Vitro Skin Irritation: Reconstructed Human Epidermis Test Method", OECD Guidelines for the Testing of Chemicals, 2015

### {Summary of Invention}

### {Technical Problem}

The MTT test is inconvenient in that, in a case where absorbance is to be measured with a predetermined dye after the disruption of cells during measurement, the three-dimensional structure of the skin model is lost. With the MTT test, in a case where the number of living cells is to be counted using only two-dimensional images one at a time, only information about the region near the surface may be acquired, and furthermore, how chemicals affect the skin from the skin surface downward in the depth direction may not be assessed correctly because cells existing at different positions in the depth direction are mixed on the same image.

The present invention has been conceived in light of the above-described circumstances, and an object thereof is to provide a method for assessing irritancy to a biological sample in which the irritancy of a chemical in the depth direction can be assessed while still preserving the three-dimensional structure of the biological sample.

### {Solution to Problem}

In order to achieve the above-described object, the present invention provides the following solutions.

One aspect of the present invention is a method for assessing irritancy to a biological sample in which the irritancy of a chemical to a three-dimensional biological sample having a plurality of layers in the depth direction is assessed, the method including: an image acquisition step of acquiring, at different depths in the biological sample, fluorescence images of a nuclei of cells in at least one group from among a living cell group and a dead cell group in the biological sample exposed to the chemical, the nuclei of cells in at least one group being labeled with a fluorescent marker; and an assessment step of calculating a ratio of the number of cells in the at least one group existing in the depth direction on the basis of the plurality of fluorescence images acquired in the image acquisition step.

According to this aspect, in the image acquisition step, fluorescence images of the nuclei of cells in at least one group of the living cell group and the dead cell group are acquired at a plurality of positions in the depth direction of the biological sample. If the chemical is not irritant to cells, the ratio of the number of nuclei of living cells (i.e., cell survival rate) in the fluorescence images becomes large, and the ratio of the number of nuclei of dead cells (i.e., cell death rate) becomes small. In contrast, if the chemical is irritant to cells, cell death takes place in the biological sample, thus leading to a decrease in the ratio of the number of nuclei of living cells in the fluorescence image, and an increase in the ratio of the number of nuclei of dead cells. Therefore, in the assessment step, it is possible to assess whether or not the chemical is irritant to the biological sample on the basis of the ratio of the number of cells in at least one group of the living cell group and the dead cell group in each of the fluorescence images. Because typical biological models contain a substantially constant, known number of cells, the total number of cells included in a biological model before a test is conducted can be acquired. The ratio of the number of living cells and/or dead cells obtained from the fluorescence images with respect to the total number of cells acquired in this manner can also serve as a criterion for irritancy assessment. Before a test is conducted, the positions and number of all cells existing in the depth direction of the biological model may be obtained in advance, as required, by observation means that does not use fluorescence, such as with a phase-contrast microscope.

In this case, irritancy of the chemical can be assessed while still preserving the three-dimensional structure of the biological sample by using fluorescence images, which can be acquired without disrupting the biological sample. In addition, by using the plurality of fluorescence images acquired at the different depths of the biological sample, irritancy of the chemical in the depth direction in the biological sample can be assessed.

In the above-described aspect, in the image acquisition step, a fluorescence image including a surface of the biological sample may be acquired, and the assessment step may further include a step of assessing a correlation between the ratio and a distance, in the depth direction, from the surface or a surface layer of the biological sample. In this case, the correlation may be assessed according to the surface shape of the biological sample on the basis of the ratio and the position in the depth direction of the biological sample.

By doing so, irritancy at each position in the depth direction of the biological sample can be assessed in more detail.

In the above-described aspect, in the image acquisition step, the fluorescence images serving as two-dimensional planes substantially parallel to the plurality of layers may be acquired, and, in the assessment step, the ratio of the cells in the two-dimensional planes may be calculated for at least one layer.

By doing so, because each of the fluorescence images includes the nuclei of cells in one layer, whether or not the chemical is irritant for each layer can be assessed even more correctly.

The above-described aspect may further include a three-dimensional image reconstruction step of reconstructing a three-dimensional image of the biological sample from the plurality of fluorescence images acquired in the image acquisition step.

By doing so, even in a case where the surface of the biological sample is not flat or the surface of the biological sample is not parallel to the fluorescence image plane, whether or not the chemical is irritant in the depth direction can be assessed correctly on the basis of the three-dimensional image.

In the above-described aspect, the assessment step may include a vertical cross-section image generation step of generating a partial three-dimensional image formed of a vertical cross-section image in a direction along the depth direction.

The vertical cross-section image includes a plurality of layers of the biological sample. Therefore, whether or not the chemical is irritant and the level of irritancy can be easily assessed for each layer on the basis of the vertical cross-section image.

In the above-described aspect, in the vertical cross-section image generation step, two or more vertical cross-section images corresponding to different positions on the fluorescence images may be generated, and, in the assessment step, the ratios in the two or more vertical cross-section images may be compared.

By doing so, the irritancy of the chemical can be assessed two-dimensionally in a direction parallel to the layers.

In the above-described aspect, in the image acquisition step, a fluorescence image of the nuclei of dead cells may be acquired, and, in the assessment step, the ratio of the number of dead cells may be calculated.

In the above-described aspect, in the assessment step, it may be determined whether the nuclei of dead cells have lost the shapes thereof on the basis of the sizes of the nuclei of dead cells in the fluorescence image.

From among cells that die due to irritation of chemicals, some die with the shapes of the nuclei being preserved, and others die with the nuclei thereof being disrupted. From among the nuclei of dead cells, the nuclei that have lost the shapes thereof as a result of disruption are indicated as large fluorescent regions in the fluorescence images, compared with nuclei preserving the shapes thereof. Therefore, a difference in how the life of cells has come to an end can be determined on the basis of the sizes of the nuclei of dead cells in the fluorescence images.

In the above-described aspect, in the image acquisition step, fluorescence images of the nuclei of living cells and the nuclei of dead cells that are fluorescence-labeled with fluorescent markers having emission wavelengths different from each other may be acquired, and, in the assessment step, the ratio of the number of living cells with respect to the sum of the number of living cells and the number of dead cells may be calculated.

By doing so, the irritancy of the chemical can be assessed on the basis of the cell survival rate.

In the above-described aspect, in the assessment step, it may be determined that the chemical is not irritant if the ratio of the number of living cells is larger than a predetermined threshold value, it may be determined that the chemical is irritant if the ratio of the number of living cells is equal to or smaller than the predetermined threshold value, and the predetermined threshold value may be set for each of the fluorescence images.

In some cases, the biological sample may contain dead cells before being exposed to the chemical, and the ratio of such dead cells not associated with the chemical differs depending on the layer. Therefore, by setting a threshold value for each fluorescence image according to the ratio of the number of dead cells that are contained in each of the layers and that are not associated with chemical treatment, the cell survival rate in each of the layers can be calculated even more correctly, and thereby, the irritancy of the chemical can be assessed even more correctly.

### {Advantageous Effects of Invention}

The present invention affords an advantage in that irritancy of a chemical in the depth direction can be assessed while still preserving the three-dimensional structure of a biological sample.

### {Brief Description of Drawings}

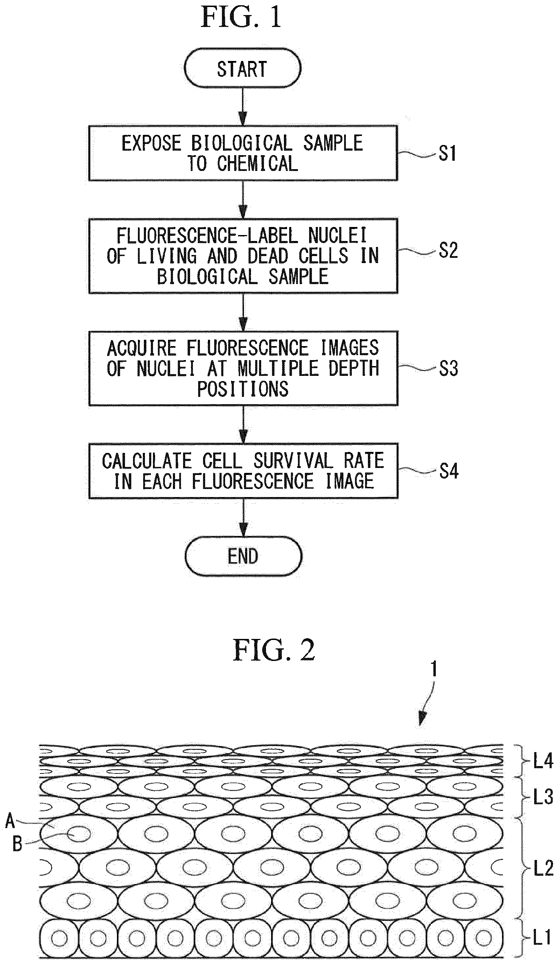

{Fig. 1}
   Fig. 1 is flowchart for illustrating a method for assessing irritancy to a biological sample according to one embodiment of the present invention.
{Fig. 2}
   Fig. 2 is a schematic diagram depicting a layer structure of a biological sample used in a method for assessing irritancy to a biological sample according to one embodiment of the present invention.
{Fig. 3}
   Fig. 3 is one example of a fluorescence image of the nuclei of living cells acquired in an example of the present invention.
{Fig. 4}
   Fig. 4 is one example of a fluorescence image of the nuclei of dead cells acquired in an example of the present invention.
{Fig. 5}
   Fig. 5 is a three-dimensional reconstructed fluorescence image showing cell nuclei of a human skin model exposed to 1% Triton-X to cause irritation in an example of the present invention.
{Fig. 6}
   Fig. 6 is a three-dimensional reconstructed fluorescence image showing cell nuclei of a human skin model exposed to 10% Triton-X to cause irritation in an example of the present invention.
{Fig. 7}
   Fig. 7 is a three-dimensional reconstructed fluorescence image showing cell nuclei of a human skin model exposed to 1% acetic acid to cause irritation in an example of the present invention.
{Fig. 8}
   Fig. 8 is a three-dimensional reconstructed fluorescence image showing cell nuclei of a human skin model exposed to 10% acetic acid to cause irritation in an example of the present invention.
{Fig. 9}
   Fig. 9 is a three-dimensional reconstructed fluorescence image showing cell nuclei of a human skin model exposed to PBS to cause irritation in an example of the present invention.
{Fig. 10}
   Fig. 10 is a three-dimensional reconstructed fluorescence image showing cell nuclei of a human skin model exposed to 5% SDS to cause irritation in an example of the present invention.
{Fig. 11}
   Fig. 11 is flowchart for illustrating a modification of the method for assessing irritancy to a biological sample in Fig. 1.

### {Description of Embodiments}

A method for assessing irritancy to a biological sample according to one embodiment of the present invention will now be described with reference to the drawings.

The method for assessing irritancy to a biological sample according to this embodiment is a method for assessing chemical-induced irritation to the skin and, as shown in Fig. 1, includes: a chemical treatment step S1 of treating a three-dimensional biological sample, simulating the skin, with a test sample containing a chemical; a fluorescence labeling step S2 of fluorescence-labeling the nuclei of the cells in the biological sample; an image acquisition step S3 of acquiring fluorescence images of the biological sample having the nuclei fluorescence-labeled; and an assessment step S4 of analyzing the fluorescence images and assessing the irritancy of the chemical.

As shown in Fig. 2, a biological sample 1 has a three-dimensional structure reconstructed by simulating the layer structure (multilayer structure including the stratum corneum, the granular layer, the stratum spinosum, and the basal layer) of the human epidermis and includes: a surface with which chemical substances and so forth come into contact as a result of chemical treatment; and a plurality of layers L4, L3, L2, and L1 that are stacked in the depth direction (up-down direction in Fig. 2) from the surface inward. At least one of the layers includes cells A. Reference sign B denotes nuclei.

As such a biological sample 1, a three-dimensional human skin model approved in the OECD Testing Guidelines (TG) 439 (In Vitro Skin Irritation: Reconstructed Human Epidermis Test Method), such as SkinEthic RHE (manufactured by SkinEthic, France) having a thickness of about 100 µm, is favorably used. Other three-dimensional human skin models that can be used in this embodiment include EpiDerm (manufactured by Kurabo Industries Ltd., Japan), LabCyte EPI-MODEL (manufactured by J-TEC, Japan), Testskin LSE (manufactured by Toyobo Co., Ltd., Japan), Episkin (manufactured by EpiskinSNC, France), and so forth. For irritancy assessment, dermal tissue may be contained in addition to the epidermis.

The human skin model may include accessory elements, such as blood vessels, lymphoduct, sebaceous glands, sweat glands, hair, and hair follicles. More specifically, such accessory elements include vascular endothelial cells, lymph duct endothelial cells, immune cells, melanocytes, dendritic cells, Langerhans cells, trichocyst cells, hair papilla cells, sebaceous gland cells, fat cells, and so forth. In addition, the biological model may include other accessory elements accompanying the organ of interest. Furthermore, the biological model may include neoplastic cells specific to some pathological conditions, such as a tumor, inflammation, polyp, or plaque.

The chemical treatment step S1 is carried out in accordance with TG439. More specifically, in the chemical treatment step S1, the test sample containing the chemical is uniformly applied to the surface of the biological sample, the biological sample is exposed to the test sample for 42 minutes (± one minute), and then the biological sample is washed with PBS (phosphate buffered saline). Examples of the chemical include an ingredient contained in cosmetics or quasi drugs, such as a surface-active agent like Triton-X and a weak acid like acetic acid. A fluid chemical may be applied to the biological sample by spraying. In the case where a substance that can exist in a gas (e.g., volatile substance, aerosol, or radioactive substance) is to be prepared as the chemical for the test, it is advisable to expose the biological sample in a gas having a certain concentration of the substance for a predetermined time period. This chemical treatment step S1 is normally carried out by a tester manually but may be carried out by an automatic dispensing device.

The biological sample, after being washed, is cultured for 42 hours under an environment of 37 °C and 5% CO₂. If the chemical is not irritant to cells, the cells will survive even after 42 hours of culturing at a high probability. In contrast, if the chemical is irritant to cells, the cells will die due to apoptosis or necrosis during the culture period.

In the chemical treatment step S1, biological tissues are exposed to a negative standard sample and a positive standard sample, instead of the test sample, to serve as a negative control and a positive control respectively, and are then cultured. The negative standard sample and the positive standard sample are preferably PBS and a 5% SDS (sodium dodecyl sulphate) solution, respectively, as recommended in TG439.

Next, in the fluorescence labeling step S2, the nuclei of the cells in the biological tissues are fluorescence-labeled with a predetermined fluorescent marker. For fluorescence labeling, for example, a first fluorescent dye that specifically labels the nuclei of living cells, and a second fluorescent dye that emits fluorescence of a wavelength different from that of the first fluorescent dye and that specifically labels the nuclei of dead cells are used. As the first and second fluorescent dyes, for example, Nuclear-ID, a cell life/death determining reagent (manufactured by Enzo Life Science), in which a dye that is membrane-permeable and that specifically labels the nuclei of living cells and a dye that is not membrane-permeable and that specifically labels the nuclei of dead cells are mixed, is used.

Alternatively, a first fluorescent marker for labeling the nuclei of both living cells and dead cells, and a second fluorescent marker that specifically labels the nuclei of only dead cells and that has an emission wavelength different from that of the first fluorescent marker may be used. As the first fluorescent marker, Hoechst33342, Hoechst33258, Draq5, Acridine Orange, and so forth are used. As the second fluorescent marker, DAPI, Propidium Iodide, ethidium bromide, and so forth are used. In this case, the irritancy of the chemical can be assessed from the ratio between the total number of cells and the number of dead cells in the assessment step S4, which will be described later.

Next, in the image acquisition step S3, fluorescence images of the nuclei of living cells and the nuclei of dead cells at different depth positions in the biological sample are acquired using a fluorescence microscope. More specifically, two-dimensional planar fluorescence images substantially parallel to each of the layers of the biological sample are acquired through sequential, two-dimensional scanning by using, for example, a confocal laser microscope, while constantly shifting the focal plane in the depth direction of the biological sample at intervals of a short distance (e.g., 1 µm to 5 µm, preferably 2 µm or 3 µm) by changing the relative distance between the biological sample and the objective lens over time along a constant optical axis, and thereafter, the acquired fluorescence images are stacked in the depth direction.

An illumination device for the fluorescence microscope may have a laser light source for generating excitation light having a wavelength in accordance with the type of the markers used for fluorescence labeling or may have a laser light source containing wavelengths other than the excitation wavelength, like white light. The objective lens is disposed above or below a culture plate for accommodating the biological sample so as to face the surface of the biological sample, and slice images (fluorescence images) including the surface of the biological sample are acquired by moving the focal plane of the objective lens up to the plane including a space slightly above the surface of the biological sample. Although the images may be acquired in the order from the surface down to a deep region or from a deep region up to the surface, it is preferable that images be sequentially acquired from the surface down to the deep region in a case where image processing is to be performed on the basis of information about the surface.

The short distance by which the focal plane is shifted is set so that all cells distributed in the depth direction can be captured, and two-dimensional fluorescence images are acquired from all layers, including the surface, on the basis of the set short distance. The use of fluorescence is preferable in that information about the life or death of cells can be correctly grasped by detecting whether or not cell nuclei preserve the shapes thereof and where cell nuclei are located, even in a case where the shapes of cell nuclei are changed due to, for example, the chemical or the outlines of cells become unclear, for example, as a result of cell membranes being disrupted. Because fluorescence can be selectively excited by changing the focal point of the excitation light individually for each depth, acquiring fluorescence images is convenient in acquiring an image at each depth. Each of the fluorescence images includes the nuclei of cells in one layer, whereby a plurality of fluorescence images in which the nuclei of living cells and the nuclei of dead cells are indicated as high-luminance regions having colors different from each other are acquired. In this manner, a highly accurate three-dimensional image suitable for image assessment can be acquired for a plurality of layers.

In the image acquisition step S3, it is not necessary to acquire fluorescence images of all layers, but it is also acceptable to acquire fluorescence images of some layers (e.g., only the stratum corneum corresponding to the surface layer, only the granular layer corresponding to the middle layer, or only the stratum spinosum or the basal layer corresponding to the deep layer) from among the plurality of layers. The image acquisition step S3 may be carried out in a culture chamber or may be carried out in a culture apparatus.

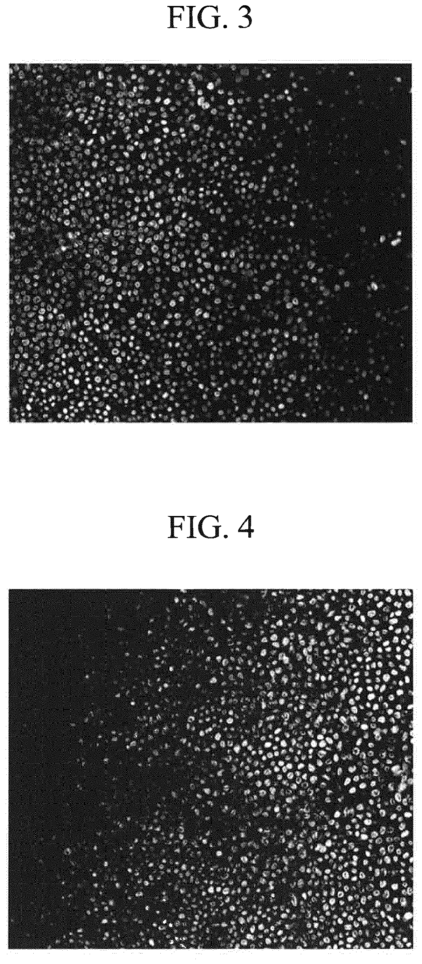

Figs. 3 and 4 show fluorescence images of the nuclei of living cells and the nuclei of dead cells, respectively. Of the three-dimensional image reconstructed from many two-dimensional slice images acquired with the confocal laser microscope, Figs. 3 and 4 each show one slice image at a depth in the midway position, illustrating images of two color channels (e.g., red channel and green channel) separated from the one slice image so that the nuclei of living cells and the nuclei of dead cells can be clearly identified.

Next, in the assessment step S4, the number of nuclei of living cells and the number of nuclei of dead cells existing in the depth direction of the biological sample are counted in each of the fluorescence images. Here, because the fluorescence images include the surface of the biological sample, correct information about the distance (depth) from the surface, which is in contact with the chemical, to an inner region of the biological sample is obtained. Furthermore, because the distance from the surface of the sample to each of the layers is predetermined in typical biological models, the distance from the surface to each of the layers can be easily identified from the images. In addition, the sizes of nuclei in the fluorescence images fall within a certain range. Therefore, the nuclei of living cells and the nuclei of dead cells in the fluorescence images can be recognized and counted on the basis of colors, luminance values, and sizes thereof.

Next, in each of the plurality of fluorescence images acquired at individual depths, the ratio of the number of nuclei of living cells (i.e., cell survival rate) with respect to the total number of all nuclei (the sum of the number of nuclei of living cells and the number of nuclei of dead cells) in a two-dimensional plane, serving as each of the fluorescence images, is calculated. Subsequently, it is determined that the chemical is not irritant if the cell survival rate in every layer or particular layers of the biological sample is larger than 50% (predetermined threshold value), and it is determined that the chemical is irritant if the cell survival rate is equal to or smaller than 50%. By doing so, it is determined whether or not the chemical is irritant for each of the depth positions of the biological sample at which the fluorescence images have been acquired. Here, a user can calculate the ratio of living cells and/or dead cells by visually measuring the images displayed on a display monitor or the images output with a printer.

Irritancy in one layer can be assessed independently by obtaining the ratio of the number of living cells existing in the depth direction with respect to the total length of that layer in the depth direction. Cell death due to, for example, aging or deterioration in a culture medium is more likely to take place or develop in deeper regions in a layer deeper than the surface or surface layer of the biological sample. In most cases, cell death taking place in an irritation test develops starting at the surface of the biological sample to which a chemical is applied. Therefore, by comparing both the ratio and the positions (distances from the surface) of living cells in the depth direction, the influence of the chemical can be assessed as being distinguished from the influences of, for example, deterioration of the biological model in the actual biological sample or a device failure of the culture apparatus. For example, if the number of living cells is reduced specifically in a deep layer away from the surface layer of the biological sample, the data can be excluded from the test results or can be separately recognized as an abnormality. On the other hand, in some cases, it may be determined that the chemical is selectively irritant (or has become more irritant) in particular layers, regardless of the distance from the surface of the biological sample. In any case, whether or not there is a correlation between irritancy of the chemical and the distance from the surface of the biological sample provides beneficial information in the irritation test. Because the surface layer including the skin surface exhibits diverse states depending on the health condition and allergic diathesis, irritancy to the surface or the surface layer of the biological sample may also affect irritancy to layers deeper than the surface layer. In contrast to this, if the irritancy to the surface or surface layer of the biological sample is not associated with irritancy to layers deeper than the surface layer, irritancy assessment involving the number of living cells on the surface or in the surface layer is not useful. Therefore, the present invention can provide a method of allowing the assessment of the number of living cells in the depth direction individually for each layer, thereby making it possible to correctly assess both the number of living cells in each layer and whether or not there is a correlation between the number of living cells and the irritancy in the surface or the surface layer of the biological sample.

The processing for the assessment step S4 described above is achieved by an irritancy assessment program executed in a computer. On the other hand, a three-dimensional fluorescence image may be displayed on the display monitor or projected, so that an observer can intuitively assess irritancy. In addition, the ratio of the number of living cells, serving as information assessed with the above-described assessment program, may be superimposed on the three-dimensional image on the display monitor individually for each layer or may be output in the form of statistical data, such as a graph.

As described above, according to the method for assessing irritancy to a biological sample of this embodiment, through three-dimensional fluorescence imaging in which a plurality of fluorescence images are acquired at constant intervals in the depth direction, the number of living cells and the number of dead cells in the biological sample can be measured to allow assessment of chemical-induced irritation to the biological sample while preserving the three-dimensional structure of the biological sample.

In particular, unlike the MTT test, the cell survival rate at any depth position from the deep layer to the surface layer of the biological sample can be calculated. Chemicals with high skin permeability and chemicals with low skin permeability have different influences on each of the layers. For example, chemicals with low skin permeability act only on the surface layer and do not act on the middle layer and the deep layer, whereas chemicals with high skin permeability act on all layers. According to this embodiment, unlike the MTT test, irritancy of the chemical can be assessed individually for each layer.

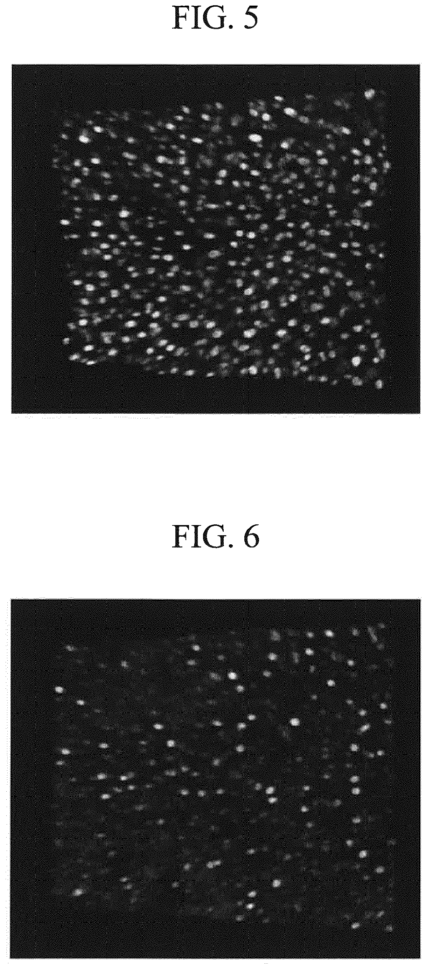

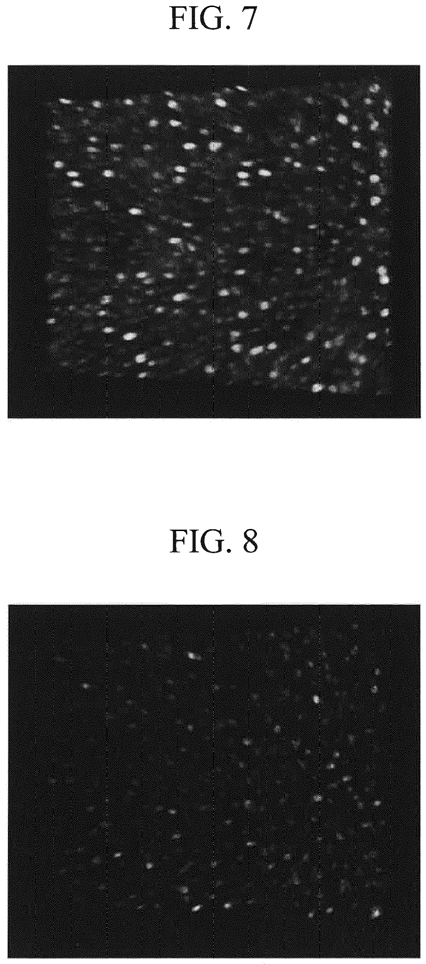

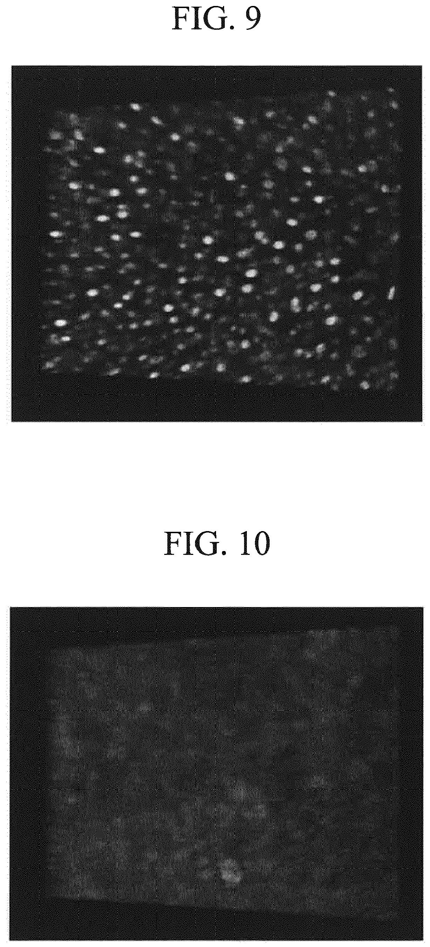

Figs. 5 to 10 each show one example of a three-dimensional image reconstructed from fluorescence images acquired according to the method for assessing irritancy to a biological sample of this embodiment. Figs. 5 and 6 show fluorescence images of biological samples exposed to 1% and 10% Triton-X, respectively. Figs. 7 and 8 show fluorescence images of biological samples exposed to 1% and 10% acetic acids, respectively. Figs. 9 and 10 show fluorescence images of biological samples exposed to PBS and a 5% SDS solution, as controls.

In the examples shown in Figs. 5 to 10, human skin models based on SkinEthic RHE were exposed to the chemicals in accordance with TG439, and the nuclei of living cells and the nuclei of dead cells were fluorescence-labeled with Nuclear-ID, a cell life/death determining reagent.

As is seen in Figs. 5 to 8, three-dimensional structures of cells, such as the nuclei, were preserved in the human skin models exposed to Triton-X, acetic acid, and PBS. In addition, from these three-dimensional images, it was found that there were regions in which both living cells and dead cells coexisted, and that dead cells were dispersed in the deep layers of the biological samples, or conversely living cells were dispersed in the surface layers. Even in the case of biological models, the surfaces of the biological samples are not always flat and may have undulations partly or entirely or have minute dimples or projections locally. Variations in these internal structures and surface shapes are common both in biological models and biological samples derived from organisms.

Furthermore, although structures such as the nuclei of dead cells were preserved in the human skin model exposed to 10% acetic acid, the structures such as the nuclei of dead cells were disrupted in the human skin model exposed to a 5% SDS solution. Such disruption of cell structures is a phenomenon that cannot be detected with the MTT test.

In the assessment step S4 of this embodiment, as the threshold value for irritancy determination, the same value (50%) is used for all fluorescence images. Instead of this, a threshold value may be set for each of the fluorescence images.

It is possible that dead cells existed in each of the layers of the biological sample before the chemical treatment step S1 is carried out. If the number of such dead cells not associated with irritation due to the chemical is counted in the cell survival rate, the irritancy of the chemical cannot be correctly assessed.

Most of the cells exposed to the negative standard sample, such as PBS, survive. Therefore, the number of dead cells not associated with irritation of the chemical is obtained by acquiring a plurality of fluorescence images of the biological sample exposed to the negative standard sample in the same manner as in the biological sample exposed to the test sample, and then measuring the number of dead cells in each of the fluorescence images. Thereafter, a threshold value can be set for each of the fluorescence images (e.g., the higher the number of dead cells not associated with irritation of the chemical is, the higher the threshold value that is set) by taking into account the obtained number of dead cells not associated with chemical-induced irritation, thereby making it possible to obtain a correct survival rate of cells in response to irritation of the chemical. By doing so, the irritancy of the chemical can be assessed even more correctly.

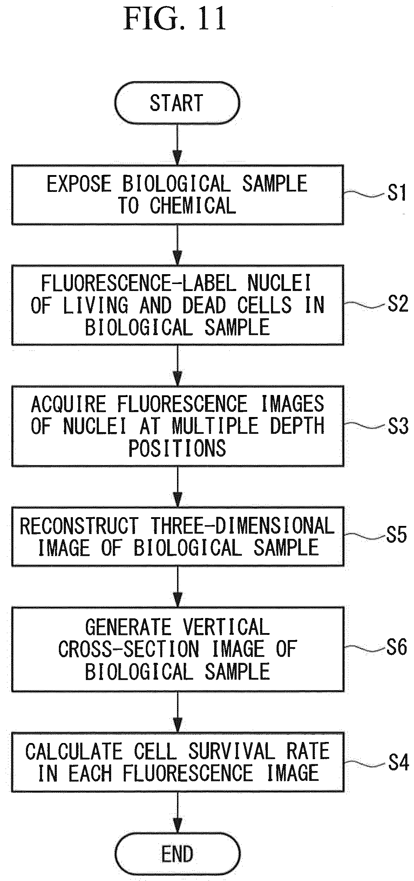

In this embodiment, the three-dimensional image reconstruction step S5 may further be included between the image acquisition step S3 and the assessment step S4, as shown in Fig. 11.

In the three-dimensional image reconstruction step S5, a three-dimensional image of the biological sample is reconstructed by stacking, in the depth direction, all of the plurality of two-dimensional fluorescence images acquired in the image acquisition step S3, such that addresses in the XY directions coincide with one another. Even in a case where the surface shape of the biological sample is not flat or the surface of the biological sample is not orthogonal to the optical axis of the objective lens, correct assessment in the depth direction can be performed according to the position of the surface by performing depth-by-depth assessment on the basis of the three-dimensional image.

In addition, the vertical cross-section image generation step S6 of generating a vertical cross-section image, which is a partial three-dimensional image, either subsequently to the three-dimensional image reconstruction step S5 or independently thereof, may be included between the image acquisition step S3 and the assessment step S4. In the vertical cross-section image generation step S6, after the three-dimensional image has been reconstructed in the three-dimensional image reconstruction step S5, a vertical cross-section image of the biological sample taken in a direction along the depth direction can be generated by extracting only a portion with the same address corresponding to a linear or rectangular region on all of the two-dimensional images constituting the three-dimensional image, as shown in Fig. 11. Without reconstructing the three-dimensional image, a vertical cross-section image of the biological sample taken in a direction along the depth direction may be independently generated by stacking portions with the same address corresponding to a linear or rectangular region on each of the two-dimensional images.

The vertical cross-section image includes a plurality of layers of the biological sample and preferably includes the surface of the biological sample. Therefore, in the assessment step S4, the cell survival rate can be calculated individually for each layer by measuring the number of nuclei of living cells and the number of nuclei of dead cells in each of the layers on the basis of the vertical cross-section image.

The above-described processing for the three-dimensional image reconstruction step S5 and/or the vertical cross-section image generation step S6 is carried out with an image processing program that is executed in an image processing apparatus or the computer. The reconstructed three-dimensional image can be rotated at a desired angle on the display monitor, and furthermore, the monitor display can be switched, as required, so as to display a vertical cross-section image at an arbitrary position on the three-dimensional image. In addition, for the generated vertical cross-section image, one or more cross sections, such as a sheet-shaped cross section, plate-shaped cross section, block-shaped cross section, and columnar cross section, can be displayed on the display monitor by specifying, in the image processing apparatus, address information to be cut out.

It is possible to additionally provide an assessment step of identifying the position and the depth that are most prone to irritation in the two-dimensional image of the surface or a deep region of the biological sample, and this can be achieved by generating two or more vertical cross-section images on the basis of two or more different addresses, displaying or measuring the generated vertical cross-section images, and comparing the two or more vertical cross-section images. Because a difference in the number of living cells between cross sections orthogonal to the surface of the biological sample obtained in this manner reflects a difference in two-dimensional chemical influence on the biological sample, it is possible to specifically assess a region that is most likely to have the highest contact density or permeation level. This is effective in a highly accurate understanding of the maximum risk of the chemical to be tested.

In this embodiment, from among the nuclei of dead cells, the number of nuclei that have been disrupted and lost their shapes may be measured as being distinguished from the number of nuclei of other dead cells.

For example, the nuclei of cells exposed to strongly acidic chemicals are disrupted and become extinct in terms of shape. In the MTT test, nuclei preserving the shapes thereof and nuclei that have lost the shapes thereof cannot be detected such that they are distinguished from each other. In a fluorescence image, on the other hand, nuclei that have lost the shapes thereof are observed as large fluorescent regions, compared with nuclei preserving the shapes thereof, and therefore, the number of nuclei that have lost the shapes thereof can be measured as being discriminated from other nuclei.

From among cells exposed to chemicals, some die with the shapes of the nuclei being preserved, and others die with their nuclei being disrupted. Information about how the life of cells has come to an end is useful in assessing the irritancy of chemicals. According to this embodiment, not only the cell survival rate but also how the life of cells comes to an end can be identified on the basis of the sizes of the nuclei of dead cells in the fluorescence images.

Although a human skin model is used as the biological sample in this embodiment, the method for assessing irritancy according to this embodiment can be applied to biological models of any other organs. If a biological sample has a tubular section, serving as a circulation organ or a digestive organ, the inner wall of the tubular section can be the surface of the biological sample. Materials for building a three-dimensional structure may be a sample prepared by three-dimensionally culturing cells using a spheroid, an organoid, or a matrix-shaped three-dimensional carrier for culturing (e.g., Matrigel (registered trademark, manufactured by Corning) and Cellbed (registered trademark, manufactured by Japan Vilene Company, Ltd.)), or may be a laminate of cell sheets prepared in such a manner.

In this embodiment, the chemical treatment step S1 is carried out under the conditions in accordance with TG439, including the type of the chemical and the treatment procedures. However, the conditions for the chemical treatment step S1 may be changed on the basis of the type of test and applicable regulations in countries where the relevant chemical is produced or sold. In addition, the chemical treatment step S1 may be carried out by affixing a sticking seal containing the chemical to the surface of the biological sample or by implanting, in the biological sample, an implant that has a biomaterial as the principal ingredient and that contains the chemical.

Furthermore, the fluorescent markers used for fluorescence labeling in the fluorescence labeling step S2 are not limited to a fluorescent dye but may be fluorescent proteins.

Although in this embodiment, the nuclei of living cells and the nuclei of dead cells are labeled with fluorescent markers having different emission wavelengths from each other, the nuclei of at least either of living cells and dead cells may be labeled. For example, only living cells may be fluorescence-labeled for assessment. In this case, the ratio of living cells at each depth can be assessed by assessing the distribution of the living cells in a three-dimensional space.

In the image acquisition step S3 of this embodiment, the three-dimensional fluorescence image may be acquired by using a method other than the method in which the confocal microscope is used. A three-dimensional image may be acquired at high speed through one or several image acquisitions, for example, by making the biological sample transparent with a transparency-inducing reagent right before image acquisition and then acquiring images of the transparent biological sample with a depth of focus including a plurality of layers of the biological sample while acquiring information about the depth and surface using the method of stereoscopic observation or phase difference image acquisition. In this case, it is advisable to separate, after the imaging of the three-dimensional image, depth-by-depth image data (e.g., a plurality of images corresponding to the case where the focal plane is shifted at intervals of the short distance) from the three-dimensional image and to make a determination as to the irritancy in the depth direction on the basis of information about the surface.

In this embodiment, the assessment step S4 may be carried out such that the ratio of the number of living cells distributed in the depth direction of each of the layers is measured by using only vertical cross-section images acquired from the three-dimensional image. In this case, it is possible to provide a system for allowing analysis of irritation tests of many kinds of chemicals having different ingredients or concentrations all at once on the same biological sample by acquiring slice-shaped or block-shaped images having one or more vertical cross sections while associating those images with address information at different sites on the surface of the biological sample.

Furthermore, the level of influence of the chemical in the depth direction can be assessed by performing image acquisition over time in the image acquisition step S3. For example, it is possible to assess in detail not only the correlation between the irritancy of the chemical and irritancy to a healthy biological model but also the correlation between the irritancy of the chemical and the therapeutic or beauty effect on an unhealthy model reflecting various pathological conditions, aging, or the like by comparing the ratio of the number of living cells between two or more time points including the time point immediately after the end of a predetermined culture period and a time point a certain amount of time after a midway point and/or the end of the culture period.

### {Reference Signs List}

1 Biological sample
L1, L2, L3, L4 Layer
A Cell
B Nucleus
S1 Chemical treatment step
S2 Fluorescence labeling step
S3 Image acquisition step
S4 Assessment step
S5 Three-dimensional image reconstruction step
S6 Vertical cross-section image generation step

## Claims

1. A method for assessing irritancy to a biological sample in which the irritancy of a chemical to a three-dimensional biological sample having a plurality of layers in a depth direction is assessed, the method comprising:
an image acquisition step of acquiring, at different depths in the biological sample, fluorescence images of a nuclei of cells in at least one group from among a living cell group and a dead cell group in the biological sample exposed to the chemical, the nuclei of cells in at least one group being labeled with a fluorescent marker; and
an assessment step of calculating a ratio of the number of cells in the at least one group existing in the depth direction on the basis of the plurality of fluorescence images acquired in the image acquisition step.

2. The method for assessing irritancy to a biological sample according to claim 1, wherein, in the image acquisition step, a fluorescence image including a surface of the biological sample is acquired, and
the assessment step further includes a step of assessing a correlation between the ratio and a distance, in the depth direction, from the surface or a surface layer of the biological sample.

3. The method for assessing irritancy to a biological sample according to claim 2, wherein, in the assessment step, the correlation is assessed according to a surface shape of the biological sample on the basis of the ratio and a position in the depth direction of the biological sample.

4. The method for assessing irritancy to a biological sample according to one of claims 1 to 3, wherein, in the image acquisition step, the fluorescence images serving as two-dimensional planes substantially parallel to the plurality of layers are acquired, and
in the assessment step, the ratio of the cells in the two-dimensional planes is calculated for at least one layer.

5. The method for assessing irritancy to a biological sample according to one of claims 1 to 4, further comprising: a three-dimensional image reconstruction step of reconstructing a three-dimensional image of the biological sample from the plurality of fluorescence images acquired in the image acquisition step.

6. The method for assessing irritancy to a biological sample according to one of claims 1 to 5, wherein the assessment step includes a vertical cross-section image generation step of generating a partial three-dimensional image formed of a vertical cross-section image in a direction along the depth direction.

7. The method for assessing irritancy to a biological sample according to claim 6, wherein, in the vertical cross-section image generation step, two or more vertical cross-section images corresponding to different positions on the fluorescence images are generated, and
in the assessment step, the ratios in the two or more vertical cross-section images are compared.

8. The method for assessing irritancy to a biological sample according to one of claims 1 to 7, wherein, in the image acquisition step, a fluorescence image of the nuclei of dead cells is acquired, and
in the assessment step, the ratio of the number of dead cells is calculated.

9. The method for assessing irritancy to a biological sample according to claim 8, wherein, in the assessment step, it is determined whether the nuclei of dead cells have lost the shapes thereof on the basis of the sizes of the nuclei of dead cells in the fluorescence image.

10. The method for assessing irritancy to a biological sample according to one of claims 1 to 9, wherein, in the image acquisition step, fluorescence images of the nuclei of living cells and the nuclei of dead cells that are fluorescence-labeled with fluorescent markers having emission wavelengths different from each other are acquired, and
in the assessment step, the ratio of the number of living cells with respect to the sum of the number of living cells and the number of dead cells is calculated.

11. The method for assessing irritancy to a biological sample according to one of claims 1 to 10, wherein, in the assessment step, it is determined that the chemical is not irritant if the ratio of the number of living cells is larger than a predetermined threshold value, it is determined that the chemical is irritant if the ratio of the number of living cells is equal to or smaller than the predetermined threshold value, and
the predetermined threshold value is set for each of the fluorescence images.
